(19) Europäisches Patentamt
European Patent Office
Office européen des brevets

(11) **EP 0 966 963 A1**

(12) **EUROPEAN PATENT APPLICATION**

(43) Date of publication:
**29.12.1999 Bulletin 1999/52**

(51) Int. Cl.$^6$: **A61K 31/40**

(21) Application number: **98109845.2**

(22) Date of filing: **29.05.1998**

(84) Designated Contracting States:
**AT BE CH CY DE DK ES FI FR GB GR IE IT LI LU MC NL PT SE**
Designated Extension States:
**AL LT LV MK RO SI**

(71) Applicants:
- **Eisenbrand, Gerhard, Prof. Dr.**
  **69120 Heidelberg (DE)**
- **CNRS,**
  **Centre National de la Recherche Scientifique**
  **75005 Paris (FR)**

(72) Inventors:
- **Eisenbrand, Gerhard, Prof. Dr.**
  **69126 Heidelberg (DE)**
- **Hössel, Ralph, Dipl.-Chem.**
  **67659 Kaiserslautern (DE)**
- **Marko, Doris, Dr.**
  **67657 Kaiserslautern (DE)**
- **Tang, Weici, Prof.Dr.**
  **67663 Kaiserslautern (DE)**
- **Meijer, Laurent, Dr.**
  **29680 Roscoff (FR)**

(74) Representative:
**Müller-Boré & Partner**
**Patentanwälte**
**Grafinger Strasse 2**
**81671 München (DE)**

(54) **Use of indigoid bisindole derivatives as CDK1 inhibitors**

(57) The present invention relates to the use of indigoid bisindole derivatives for the manufacture of a medicament for inhibiting the cyclin dependent kinase ATP:Proteinphosphotransferase p34$^{cdc2}$ (CDK1).

Fig. 1

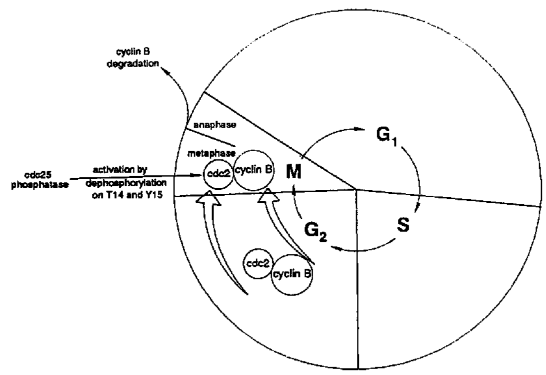


EP 0 966 963 A1

## Description

The present invention relates to the use of indigoid bisindole derivatives for the manufacture of a medicament for inhibiting the cyclin dependent kinase ATP:Proteinphosphotransferase $p34^{cdc2}$ (CDK1).

Indigoid bisindoles comprise a spectrum of natural dye stuffs. Many of these can be obtained from plants. Accordingly, indirubine, indigo and isoindigo are natural products which can be obtained from different plants: namely, Baphicacanthus cusia (Acanthaceae), Indigofera suffruticosa (Fabaceae), Isatis indigotica (Brassicaceae) and others. Indican, a glycoside which is found in plants, gives glucose and 3-hydroxyindole due to acidic or enzymatic hydrolysis. 3-Hydroxyindole is converted by air-oxidation into indigo and its isomers. Indigo naturalis (Chinese: quingdai) is the natural blue dye obtained from plant material, e.g. Isatis indigotica (Brassicaceae). Indirubine, an isomer of indigo, can be found in Indigo naturalis in an amount of up to 60% (Falbe J. & Regitz M., Römpp Chemie Lexikon (1992), 9. Aufl., Stuttgart, Georg Thieme Verlag). It occurs also in Isatis tinctoria in an amount of up to 5% which is indigenous to Central Europe (Gelius R., Z. Chem., 20, (1980), 340-341).

Indigo naturalis is reported to be used in traditional Chinese medicine as a hemostatic, antipyretic, anti-inflammatory and sedative agent in the treatment of bacterial and viral infections. Antileukemic effects of Indigo naturalis have also been reported, with indirubine being the effective principle (Ji X. et al., Acta Pharm. Sin., 16, (1981), 146-148; Gan W. J. et al., J. Hematol., 6, (1985), 611-613). Furthermore, derivatives of indirubine are known for a long time as dyes of low persistence.

Few, structurally quite different types of $p34^{cdc2}$ inhibitors have been described up to now. They are either of natural origin or derived from natural compounds and show varying degrees of inhibitory activities. Examples are Staurosporine, an alkaloid from Streptomyces sp, Butyrolactone-I from Aspergillus terreus var., Flavopiridol, a novel promising anti-tumour agent derived by partial synthesis from a parent structure found in the indian plant Dysoxylum binectariferum, 9-Hydroxyellipticin from the plants Ochrosia elliptica and Ochrosia acuminata, the purine derivatives olomoucine, roscovitine and isopentenyladenine and some peptides. The mechanism of these compounds is based on competitive inhibition of ATP binding (Meijer L., Trends in Cell Biology, 6, (1996), 393-397).

For determination of the IC 50-values: $p34^{cdc2}$/cyclin B was purified from M phase starfish (Marthasterias glacialis) oocytes by affinity chromatogaphy on $p9^{CKShs1}$-Sepharose beads, from which it was eluted by free $p9^{CKShs1}$ as described (Meijer et al., Eur. J. Biochem., 243, (1997), 527-536). It was assayed with 1 mg histone H1 (Sigma type III-S)/ml, in the presence of 15 μM [$y$-$^{32}$P] ATP (3,000 Ci/mmol; 1mCi/ml) in a final volume of 30 μl. After 10 min. incubation at 30°C, 25 μl aliquots of supernatant were spotted onto 2.5 x 3 cm pieces of Whatman P81 phosphocellulose paper, and after 20 sec., the filters were washed five times (for at least 5 min. each time) in a solution of 10 ml phosphoric acid/liter of water. The wet filters were transferred into 6 ml plastic scintillation vials, 5 ml ACS (Amersham) scintillation fluid was added and the radioactivity measured in a Packard scintillation counter. The kinase activity was expressed in pmoles phosphate incorporated into histone H1/10 min incubation or in % of maximal activity.

The strongest inhibitors of $p34^{cdc2}$ are derivatives of staurosporine ($IC_{50}$ = 0,003-0,03 μM). The selectivity of these inhibitors is, however, rather poor. They also show more or less potent inhibitory activity to quite a wide range of cellular kinases. Table 1 shows the specifity of known chemical inhibitors of cyclin-dependent kinases ($IC_{50}$-values given in μM) (cf. Meijer L., Trends in Cell Biology, 6, (1996), 393-397).

Tab. 1

| Enzyme | Stau-rosporine | UCN-01 | Butyrolac-tone-I | Flavopiridol | Olomoucin | Roscovitine | 9-Hydroxyellipticine |
|---|---|---|---|---|---|---|---|
| CDK1 | 0.003-0.009 | 0.031 | 0.60 | 0.40 | 7 | 0.65[a] | ca. 1 |
| CDK2 | 0.007 | 0.030 | 1.50 | 0.40 | 7 | 0.70 | ND |
| CDK4 | < 10.000 | 0.032 | no effect | 0.40 | > 1000 | > 100 | ND |
| MAPK | 0.020 | 0.910 | 94 | ND | 30 | 30 | ND |
| PKA | 0.008 | ND | 260 | 145 | > 2000 | > 1000 | ND |
| PKG | 0.009 | ND | ND | 6 | > 2000 | > 1000 | ND |
| PKC | 0.005 | 0.007 | 160 | ND | > 1000 | > 100 | ND |
| Tyrosinekinase | 0.006 (EGF-R) 0.006 (Src) | ND | > 590 (EGF-R) | 25 (EGF-R) | 440 (EGF-R) | 70 (I-R) | ND |
| CDK: cyclin-dependent kinase; EGF-R: epidermal growth factor receptor tyrosine kinase; I-R: insulin receptor-tyrosine kinase; MAPK: mitogene-activated protein kinase; ND: not determined; PKA: cAMP-dependent protein kinase; PKG: cGMP-dependent protein kinase. | | | | | | | |

[a]$IC_{50}$-value for racemic mixture; $IC_{50}$ for (R)-roscovitin is 0.45 μM.

Cyclins and cyclin dependent kinases (CDK) have an essential role for driving the cell through the cell cycle (cell division cycle, cdc). During the cell division cycle, oscillations in concentrations and activities of cyclins are observed. This applies, e.g. to cyclins D and E in the so-called G1-phase of the cell cycle and to cyclinA (S- and M-Phase) and cyclinB (G2- and M-Phase).

The cyclin dependent kinases are activated by association with a member of the cyclin-family. Up to now, eight human CDKs have been described: CDK1 ( = $p34^{cdc2}$) CDK2 to CDK8. CDK-proteins consist of a catalytic subunit and a regulatory subunit, the cyclins (Meijer et al., Eur. J. Biochem., 243, (1997), 527-536). Every step of the cell division cycle is regulated by specific CDK/cyclin complexes which ascertain a strict control. Important checkpoints are at the transition from G1 phase to S phase and from G2 phase to M phase (Pines J., Cancer Biology, 6, (1995), 63-72). The $p34^{cdc2}$/cyclinB complexes are important components at the $G_2$-M-checkpoint.

Fig. 1 shows the points of action of the cyclin CDK complex cdc2/cyclinB in the cell divsion cycle (M = mitosis, cell division; G = gap; S = synthesis; interphase = $G_1 + S + G_2$).

The cdc2/cyclinB complexes are the primary active protein kinases in mitosis. They accumulate in an inactive state in the cytoplasm in interphase cells, and are then rapidly activated by cdc25 phosphatase and translocated into the nucleus at the beginning of mitosis (Pines J., Hunter J., Cell Biol., 115(1), (1991),1-17). CyclinB is degraded at the metaphase-anaphase transition, inactivating cdc2, which is necessary for exit from mitosis (Glotzer et al., Nature, 349(6305), (1991), 132-138; Murray A.W., Nature 339(6222), (1989), 280-286; Surana U. et al., Cell, 65(1), (1991), 145-161). Multiple changes of CDK proteins and their regulators are associated with the development of human tumours (Cordon-Cardo C., Am. J. Pathol., 147(3), (1995), 545-560).

Thus, the technical problem underlying the present invention is to provide new inhibitors for the cyclin dependent kinase ATP:Proteinphosphotransferase $p34^{cdc2}$ (CDK1) which exhibit a high selectivity as well as high efficiency compared to the inhibitors known in the art.

The solution to the above technical problem is achieved by the embodiments characterized in the claims.

In particular, the present invention relates to the use of indigoid bisindole derivatives for the manufacture of a medicament for inhibiting the cyclin dependent kinase ATP:Proteinphosphotransferase $p34^{cdc2}$ (CDK1) in mammals, preferably in man. Preferably, the indigoid bisindole derivatives are selected from indigo derivatives, isoindigo derivatives or indirubine derivatives.

In a preferred embodiment of the present invention, the indirubine derivate is a compound having the general formula (I)

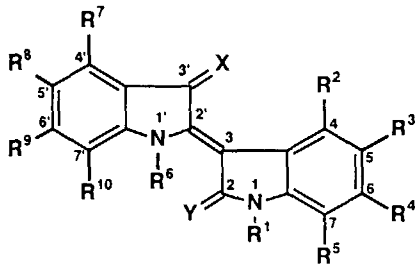

(I)

wherein the groups $R^1$ and $R^6$ can be the same or different and represent a hydrogen atom; a halogen atom; a hydroxy group; a methylenehydroxy group; a straight-chain or branched-chain alkyl group having 1 to 18 carbon atoms; a cycloalkyl group having 3 to 7 carbon atoms which can comprise one or more heteroatoms; a substituted or unsubstituted aryl group which can comprise one or more heteroatoms; a mono-, di- or trialkylsilyl group having 1 to 6 carbon atoms independently of each other in each instance in the straight-chain or branched-chain alkyl group; a mono-, di- or triarylsilyl group with substituted or unsubstituted aryl groups independently of each other in each instance; an aralkyl group; a trifluoromethyl group; a -COM group; a -COOM group; a - $CH_2COOM$ group, wherein M is hydrogen, a straight-chain or branched-chain alkyl group having 1 to 18 carbon atoms which can additionally carry one or more hydroxy and/or amino groups, or an aryl group which can comprise one or more heteroatoms and can be substituted with one or more halogen atoms, one or more alkyl groups or one or more alkoxy groups; a $-NR^{11}R^{12}$ group, wherein $R^{11}$ and $R^{12}$ can be the same or different and represent a hydrogen atom, a straight-chain or branched-chain alkyl group having 1 to 18 carbon atoms which can additionally carry one or more hydroxy and/or amino groups, a substituted or unsubstituted aryl group which can comprise one or more heteroatoms, or an acyl group; a methyleneamino group $-CH_2-NR^{11}R^{12}$, wherein $R^{11}$ and $R^{12}$ have the above definitions; a benzyl group, wherein the benzene nucleus can comprise one or more heteroatoms; a methylenecycloalkyl group having 3 to 7 carbon atoms which can comprise one or more heteroatoms; a physiological amino acid residue bound to the nitrogen as an amide; an O-glycoside or a N-glycoside, wherein the glycoside is selected from monosaccharides or disaccharides; or a methylenesulfonate group; $R^2$, $R^3$, $R^4$, $R^5$, $R^7$, $R^8$, $R^9$ and $R^{10}$ can be the same or different and represent a hydrogen atom; a halogen atom; a hydroxy group; a nitroso group; a nitro group; an aryloxy group; an alkoxy group; a straight-chain or branched-chain alkyl group having 1 to 18 carbon atoms which can additionally carry one or more hydroxy and/or amino groups; a substituted or unsubstituted aryl group which can comprise one or more heteroatoms; a cycloalkyl group having 3 to 7 carbon atoms which can comprise one or more heteroatoms; an aralkyl group; a trifluoromethyl group; a -COM group; a -COOM group; a - $CH_2COOM$ group, wherein M is hydrogen, a straight-chain or branched-chain alkyl group having 1 to 18 carbon atoms which can additionally carry one or more hydroxy and/or amino groups, or an aryl group which can comprise one or more heteroatoms and can be substituted with one or more halogen atoms, one or more alkyl groups or one or more alkoxy groups; a $-NR^{11}R^{12}$ group, wherein $R^{11}$ and $R^{12}$ can be the same or different and represent a hydrogen atom, a straight-chain or branched-chain alkyl group having 1 to 18 carbon atoms which can additionally carry one or more hydroxy and/or amino groups, a substituted or unsubstituted aryl group which can comprise one or more heteroatoms, or an acyl group, or $R^{11}$ and $R^{12}$ form together a ring having 2 to 6, optionally substituted, $CH_2$ groups; a benzyl group, wherein the benzene nucleus can comprise one or more heteroatoms; a hydroxylamino group; a phosphate group; a phosphonate group; a sulfate group; a sulfonate group; a sulfonamide group, wherein the nitrogen atom can be independently substituted by a hydrogen atom, a straight-chain or branched-chain alkyl group having 1 to 18 carbon atoms which can additionally carry one or more hydroxy and/or amino groups, a substituted or unsubstituted aryl group or wherein the nitrogen atom is part of a cycloalkyl group having 3 to 7 carbon atoms which can comprise one or more heteroatoms; an azo group $-N = N-R^{13}$, in which $R^{13}$ represents an aromatic system which can be substituted by one or more carboxyl groups, phosphoryl groups or sulfonate groups; or a O-glycoside or a N-glycoside, wherein the glycoside is selected from monosaccharides or disaccharides; or $R^1$ and $R^5$, and $R^6$ and $R^{10}$, respectively, form independently from each other a ring together having 1 to 4, optionally substituted, $CH_2$ groups; and X and Y can be the same or different and represent an oxygen atom; a sulfur atom; a selenium atom; a tellurium atom; a $NR^{14}$ group in which the group $R^{14}$ represents a hydrogen atom, a straight-chain or branched-chain alkyl group having 1 to 18 carbon atoms which can be substituted by one or more carboxyl groups, phosphoryl groups or sulfonate groups, a substituted or unsubstituted aryl group which can comprise one or more heteroatoms, an aralkyl group, or a sulfonate group; or a $NOR^{14}$ group, wherein the group $R^{14}$ has the above definitions.

With respect to the benzene nuclei constituting the indirubine derivates of the the above general formula (I) one or more ring atoms can be replaced by nitrogen atoms. Further, the indirubine derivatives having the above general

formula (I) can also be bound to a polyathyleneglycolester or a polyethyleneglycolether by ester bondings or ether bondings, respectively.

In another embodiment of the present invention, the isoindigo derivate is a compound having the general formula (II)

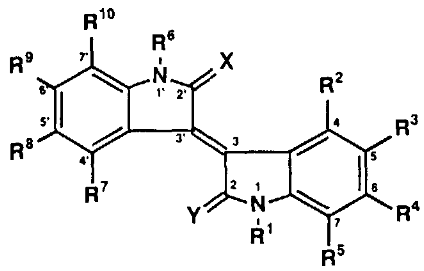

(II)

wherein $R^1$ to $R^{14}$ and X and Y have the above definitions.

With respect to the benzene nuclei constituting the isoindigo derivates of the above general formula (II) one or more ring atoms can be replaced by nitrogen atoms. Further, the isoindigo derivatives having the above general formula (II) can be bound to a polyethyleneglycolester or a polyethyleneglycolether by ester bondings or ether bondings, respectively.

In a further embodiment of the present invention, the indigo derivate is a compound having the general formula (III)

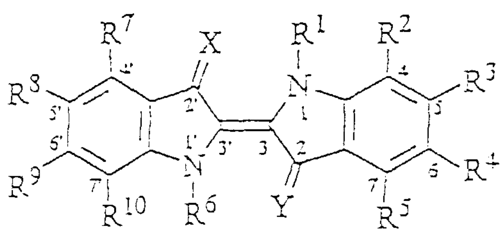

(III)

wherein $R^1$ to $R^{14}$ and X and Y have the above definitions.

With respect to the benzene nuclei constituting the indigo derivates of the above general formula (III) one or more ring atoms can be replaced by nitrogen atoms. Further, the indigo derivatives having the above general formula (III) can be bound to a polyethyleneglycolester or a polyethyleneglycolether by ester bondings or ether bondings, respectively.

The above indigoid bisindole derivatives having the general formulas (I), (II) or (III) can also be in the form of their physiologically acceptable salts.

In the search for new selective inhibitors of cellular signalling pathways, it has surprisingly been found that indigoid bisindole derivatives are highly selective inhibitors of the enzyme complex $p34^{cdc2}$/cyclinB. An inhibition of $p34^{cdc2}$-kinase by indigoid bisindole derivatives is not described in the prior art. Surprisingly, it has turned out that indigoid bisindole derivatives are both highly selective and highly effective inhibitors of cdc2-kinase, showing $IC_{50}$-values with the isolated enzyme down to submicromolar range.

Based on the above mentioned selective inhibitory potency down to the nanomolar range, indirubine derivatives can be used for analytical biochemistry, especially for the study of cell cycle effects. Furthermore, these compounds can be used for the treatment of diseases in patients, which are connected to the loss of proliferation control

without any restriction to these potential areas of application. These include cancers, psoriasis, cardiovascular diseases (stenosis, restenosis) (Brooks et al., J. Biol. Chem., 272, (1997), 29207-29211), infectious diseases (unicellular parasites (Trypanosoma, Toxoplasma, Plasmodium, etc.), fungi, etc.), nephrology (glomerulonephritis: Pippin et al., J. Clin. Invest., 100, (1997), 2512-2520), neurodegenerative disorders such as Alzheimer disease (Imahori, K., Uchida T., J. Biochem., 121, (1997), 179-188), viral infections such as cytomegalovirus (Bresnahan W. A. et al., Virology 231,(1997), 239-247) and HIV (Mancebo H.S.Y. et al., Genes & Dev., 11, (1997), 2633-2644).

The present invention is explained further by the following examples:

Example 1: Synthesis of indirubine

To a solution of 0.42 g (2.4 mmol) of indoxyl acetate in 20 ml methanol under argon 0.35 g (2.4 mmol) of isatin and 0.55 g (5.2 mmol) of sodium carbonate are added. The mixture is stirred for 30 min at ambient temperature. After 24 h standing at ambient temperature, the reaction mixture is filtered off. The precipitate is washed with little methanol and water until the filtrate shows a neutral pH. Residual water is removed by storage in an evacuated exsiccator over potassium hydroxide. Recrystallisation from ethanol or pyridine gives deep purple crystals (Russell G.A., Kaupp G. (1969), J. Am. Chem. Soc., 91, 3851-9, modified).

Yield: 0.51 g (81%), fine, deep-purple needles, Fp: 341-343°C
CHN-analysis: ($C_{16}H_{10}N_2O_2$); MW: 262,26 g/mol; calc.: 73.3% C, 3.8% H, 10.7% N; found: 73.2% C, 4.0% H, 10.6% N
mass spectrum: m/z = 262: ($M^+$, 100%), 234: (43%), 205 (25%), 158 (3%), 131 (4%), 103 (7%), 76 (3%)
$^1$H-NMR and $^{13}$C-NMR-spectrum are in accordance with the proposed structure.
IR-spectrum: 3340 $cm^{-1}$: $\nu$ (N-H), 1710 $cm^{-1}$: $\nu$ (3'-C = O), 1650 $cm^{-1}$: $\nu$ (2-C = O), 1590 $cm^{-1}$: $\nu$ (C = C, aryl), 1450 $cm^{-1}$: $\nu$ (C = C, aryl), 745 $cm^{-1}$: $\nu$ (aryl with four neighbouring H-atoms).
UV/Vis-spectrum (DMSO): 290 nm, 363 nm, 383 nm (shoulder), 551nm

Essentially the same synthetic procedere was applied for the following Examples 2 to 9 and 11 and 12:

Example 2: 5-Iodoindirubine

Yield: 80%, fine, deep-purple needles, Fp: 334-335°C (decomposition);
CHN-analysis ($C_{16}H_9IN_2O_2$); MG = 388.16 g/mol; calc.: 49.5% C, 2.3% H, 7.2% N; found.: 49.7% C, 2.5% H, 7.1% N;
Mass spectrum: 388 ($M^+$, 100%), 360 (3%), 269 (9%), 261 (6%), 233 (16%), 205 (16%), 128 (1%);
$^1$H-NMR- and $^{13}$C-NMR-spectrum are in accordance with the proposed structure.
UV/Vis-spectrum (DMSO): 370 nm, 386 nm (shoulder), 555 nm.

Example 3: 5-Bromoindirubine

Yield: 70%, fine, deep-purple needles;
CHN-analysis ($C_{16}H_9BrN_2O_2$); MG = 341.16 g/mol, calc.: 56.3% C, 2.7% H, 8.2% N; found 56.4% C, 2.7% H, 8.2% N;
Mass spectrum: 342($M^+$, 100%), 340 ($M^+$, 99%), 314 (18%), 262 (64%), 233 (34%), 205 (81%), 177 (10%);
$^1$H-NMR- and $^{13}$C-NMR-spectrum are in accordance with the proposed structure.

Example 4: 5-Chloroindirubine

Yield: 95%, fine, deep-purple needles;
CHN-analysis ($C_{16}H_9ClN_2O_2$); MG = 296.70 g/mol; calc.: 49.5% C, 2.3% H, 7.2% N; found: 49.7% C, 2.5% H, 7.1% N;
Mass spectrum: m/z = 296 ($M^+$, 100%), 268 (39%), 239 (8%), 233 (35%), 205 (50%), 177 (7%), 153 (6%), 137 (7%), 77 (7%), 120 (4%), 102 (6%), 77 (7%).

$^1$H-NMR- and $^{13}$C-NMR-spectrum are in accordance with the proposed structure.

Example 5: 5-Fluoroindirubine

Yield: 92%, fine, deep-purple needles;
CHN-analysis ($C_{16}H_9FN_2O_2$), MG = 280.25 g/mol, calc.: 68.6% C, 3.2% H, 9.9% N; found: 68.0%C, 3.2% H, 9.9% N;
Mass spectrum: m/z = 281 ($M^+$ +$H^+$, 19%), 280 ($M^+$, 100%), 252 (73%), 223 (32%), 176 (6%), 140 (7%), 121 (13%), 94 (4%), 76 (12%), 77 (7%), 57 (4%), 44(15%).
$^1$H-NMR- and $^{13}$C-NMR-spectrum are in accordance with the proposed structure.

Example 6: 5-Methylindirubine:

Yield: 92%, fine, deep-purple needles;
CHN-analysis ($C_{17}H_{12}N_2O_2$), MG = 276.28 g/mol, calc.: 73.9% C, 4.4% H, 10.1% N; found: 73.8%C, 4.3% H, 10.2% N;
Mass spectrum: m/z = 276 ($M^+$, 100%), 261 (10%), 248 (47%), 247 (53%), 220 (6%), 219 (18%), 205 (7%), 171 (4%), 165 (10%), 138 (4%), 133 (15%), 104 (7%), 77 (7%);
$^1$H-NMR- and $^{13}$C-NMR-spectrum are in accordance with the proposed structure.

Example 7: 5-Nitroindirubine

Yield: 88%, fine, deep-purple needles;
CHN-analysis ($C_{16}H_9N_3O_4$), MG = 307.26 g/mol; calc.: 62.5% C, 3.0% H, 13.7% N; found: 62.4%C, 3.0% H, 13.3% N;
Mass spectrum: m/z = 307 ($M^+$, 5%), 276 (10%), 262 (100%), 234 (23%), 205 (22%), 158 (6%), 131 (10), 104 (19%), 76 (12%), 50 (6%).
$^1$H-NMR- and $^{13}$C-NMR-spectrum are in accordance with the proposed structure.

Example 8: 5'-Bromoindirubine

Yield: 92%, fine, deep-purple needles;
CHN-analysis ($C_{16}H_9BrN_2O_2$), MG = 341.16 g/mol, calc.: 56.3% C, 2.7% H, 8.2% N; found: 55.7%C, 2.5% H, 8.0% N.
$^1$H-NMR- and $^{13}$C-NMR-spectrum are in accordance with the proposed structure.

Example 9: 5,5'-Dibromoindirubine

Yield: 94%, fine, deep-purple needles;
CHN-analysis ($C_{16}H_8Br_2N_2O_2$), MG = 420.06 g/mol; calc.: 45.7% C, 1.9% H, 6.7% N; found: 45.8% C, 2.0% H, 6.4% N.
$^1$H-NMR-spectrum is in accordance with the proposed structure.

Example 10: Indirubine-3'-oxime

Indirubine-3'-oxime was synthesized by reaction of indirubine with hydroxylamine hydrochloride in a pyridine solution (Farbwerke vorm. Meister Lucius & Brüning in Hoechst a.M., Patentschrift des Reichspatentamtes Nr. 283726 (1913)). $^{13}$C-NMR-spectroscopy revealed the location of the hydroxyimino residue in 3'-Position ($\delta$(C2) = 171.05 ppm;

δ(C3') = 145.42 ppm; DMSO-$d_6$, RT)

Yield: 90 %, red crystals;
CHN-analysis ($C_{16}H_{11}N_3O_2$), MG = 277.30g/mol; calc.: 69.3% C, 4.0% H, 15.2 % N; found: 69.0% C, 4.0% H, 14.9% N;
$^1$H-NMR- and $^{13}$C-NMR-spectrum are in accordance with the proposed structure.

Example 11: Indirubine-5-sulfonic acid

Yield: 76%, crystalline, deep-purple substance;
Mass spectrum: 388 ($M^+$, 100%), 360 (3%), 269 (9%), 261 (6%), 233 (16%), 205 (16%), 128 (1%).
$^1$H-NMR- and $^{13}$C-NMR-spectrum are in accordance with the proposed structure.

Table 2 shows the structures of the compunds of Examples 1 to 11.

Tab. 2:

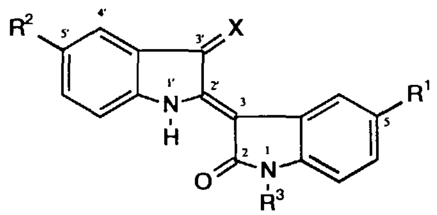

| Example | compound | $R^1$ | $R^2$ | $R^3$ | X |
|---|---|---|---|---|---|
| 1 | Indirubine | H | H | H | O |
| 2 | 5-Iodoindirubine | I | H | H | O |
| 3 | 5-Bromoindirubine | Br | H | H | O |
| 4 | 5-Chloroindirubine | Cl | H | H | O |
| 5 | 5-Fluoroindirubine | F | H | H | O |
| 6 | 5-Methylindirubine | $CH_3$ | H | H | O |
| 7 | 5-Nitroindirubine | $NO_2$ | H | H | O |
| 8 | 5'-Bromoindirubine | H | Br | H | O |
| 9 | 5,5'-Dibromoindirubine | Br | Br | H | O |
| 10 | Indirubine-3'-oxime | H | H | H | NOH |
| 11 | Indirubine-5-sulfonic acid | $SO_3H$ | H | H | O |

Table 3 shows the specificity of the compounds of Examples 1 to 11 ($IC_{50}$-values given in μM) to inhibit cdc2 kinase in comparison to other cellular kinases.

Tab. 3

| Example | cdc2 | cdc25 | PKA | PKC |
|---|---|---|---|---|
| 1 | 2 | 150 | no Effect | no Effect |
| 2 | 0.3 | 130 | no Effect | no Effect |
| 3 | 0.35 | 32 | ND | ND |
| 4 | 0.40 | 55 | ND | ND |
| 5 | 2.0 | 70 | ND | ND |
| 6 | 0.8 | 60 | ND | ND |
| 7 | 2.2 | 60 | ND | ND |
| 8 | 6.5 | ND | ND | ND |
| 9 | 7 | ND | ND | ND |
| 10 | 0.18 | ND | ND | ND |
| 11 | 0.055 | 110 | ND | ND |
| ND: not dedected; PKA: cAMP-dependent protein kinase; PKC: $Ca^{2+}$- dependent protein kinase | | | | |

Example 12: Isoindigo

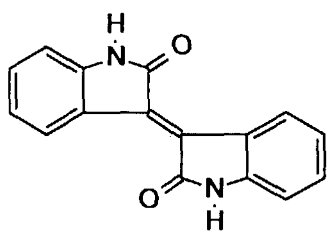

Isoindigo was synthesized by reaction of oxindole with isatin in acetic acid with addition af hydrochloric acid (Wahl A., Bayard P., Comptes Rendues Hebdomadaires des Seances de L'Academie des Sciences, 148, (1909), 716-719).

Yield: 84%, crystalline, brown substance;
CHN-analysis ($C_{16}H_{10}N_2O_2$), MG = 262.26 g/mol; calc.: 73.3% C, 3.8% H, 10,7% N; found: 73.0% C, 3.8% H, 10.9% N;
Mass spectrum: m/z = 262 ($M^+$, 100%), 234 (85%), 220 (5%), 205 (18%), 190 (4%), 177 (5%), 151 (5%), 132 (17%), 103 (6%), 76 (4%), 32 (26%).
$^1H$-NMR- and $^{13}C$-NMR-spectrum are in accordance with the proposed structure.

Isoindigo shows an $IC_{50}$-value of 80µM for the $p34^{cdc2}$/cyclinB complex.

**Claims**

1. Use of indigoid bisindole derivatives for the manufacture of a medicament for inhibiting the cyclin dependent kinase ATP:Proteinphosphotransferase $p34^{cdc2}$ (CDK1).

2. Use according to claim 1, wherein the indogoid bisindole derivatives are selected from indigo derivatives, isoindigo derivatives or indirubine derivatives.

3. Use according to claim 2, wherein the indirubine derivate is a compound having the general formula (I)

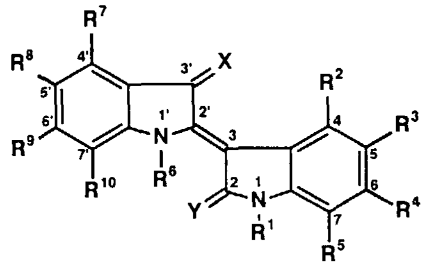

(I)

wherein the groups $R^1$ and $R^6$ can be the same or different and represent a hydrogen atom; a halogen atom; a hydroxy group; a methylenehydroxy group; a straight-chain or branched-chain alkyl group having 1 to 18 carbon atoms; a cycloalkyl group having 3 to 7 carbon atoms which can comprise one or more heteroatoms; a substituted or unsubstituted aryl group which can comprise one or more heteroatoms; a mono-, di- or trialkylsilyl group having 1 to 6 carbon atoms independently of each other in each instance in the straight-chain or branched-chain alkyl group; a mono-, di- or triarylsilyl group with substituted or unsubstituted aryl groups independently of each other in each instance; an aralkyl group; a trifluoromethyl group; a -COM group; a -COOM group; a $-CH_2COOM$ group, wherein M is hydrogen, a straight-chain or branched-chain alkyl group having 1 to 18 carbon atoms which can additionally carry one or more hydroxy and/or amino groups, or an aryl group which can comprise one or more heteroatoms and can be substituted with one or more halogen atoms, one or more alkyl groups or one or more alkoxy groups; a $-NR^{11}R^{12}$ group, wherein $R^{11}$ and $R^{12}$ can be the same or different and represent a hydrogen atom, a straight-chain or branched-chain alkyl group having 1 to 18 carbon atoms which can additionally carry one or more hydroxy and/or amino groups, a substituted or unsubstituted aryl group which can comprise one or more heteroatoms, or an acyl group; a methyleneamino group $-CH_2-NR^{11}R^{12}$, wherein $R^{11}$ and $R^{12}$ have the above definitions; a benzyl group, wherein the benzene nucleus can comprise one or more heteroatoms; a methylenecycloalkyl group having 3 to 7 carbon atoms which can comprise one or more heteroatoms; a physiological amino acid residue bound to the nitrogen as an amide; an O-glycoside or a N-glycoside, wherein the glycoside is selected from monosaccharides or disaccharides; or a methylenesulfonate group; $R^2$, $R^3$, $R^4$, $R^5$, $R^7$, $R^8$, $R^9$ and $R^{10}$ can be the same or different and represent a hydrogen atom; a halogen atom; a hydroxy group; a nitroso group; a nitro group; an aryloxy group; an alkoxy group; a straight-chain or branched-chain alkyl group having 1 to 18 carbon atoms which can additionally carry one or more hydroxy and/or amino groups; a substituted or unsubstituted aryl group which can comprise one or more heteroatoms; a cycloalkyl group having 3 to 7 carbon atoms which can comprise one or more heteroatoms; an aralkyl group; a trifluoromethyl group; a -COM group; a -COOM group; a $-CH_2COOM$ group, wherein M is hydrogen, a straight-chain or branched-chain alkyl group having 1 to 18 carbon atoms which can additionally carry one or more hydroxy and/or amino groups, or an aryl group which can comprise one or more heteroatoms and can be substituted with one or more halogen atoms, one or more alkyl groups or one or more alkoxy groups; a $-NR^{11}R^{12}$ group, wherein $R^{11}$ and $R^{12}$ can be the same or different and represent a hydrogen atom, a straight-chain or branched-chain alkyl group having 1 to 18 carbon atoms which can additionally carry one or more hydroxy and/or amino groups, a substituted or unsubstituted aryl group which can comprise one or more heteroatoms, or an acyl group, or $R^{11}$ and $R^{12}$ form together a ring having 2 to 6, optionally substituted, $CH_2$ groups; a benzyl group, wherein the benzene nucleus can comprise one or more heteroatoms; a hydroxylamino group; a phosphate group; a phosphonate group; a sulfate group; a sulfonate group; a sulfonamide group, wherein the nitrogen atom can be independently substituted by a hydrogen atom, a straight-chain or branched-chain alkyl group having 1 to 18 carbon atoms which can additionally carry one or more hydroxy and/or amino groups, a substituted or unsubstituted aryl group or wherein the nitrogen atom is part of a cycloalkyl group having 3 to 7 carbon atoms which can comprise one or more heteroatoms; an azo group $-N = N-R^{13}$, in which $R^{13}$ represents an aromatic system which can be substituted by one or more carboxyl groups, phosphoryl groups or sulfonate groups; or a O-glycoside or a N-glycoside, wherein the glycoside is selected from monosaccharides or disaccharides; or $R^1$ and $R^5$, and $R^6$ and $R^{10}$, respectively, form independently from each other a ring together having 1 to 4, optionally substituted, $CH_2$ groups; and X and Y can be the same or different and represent an oxygen atom; a sulfur atom;

a selenium atom; a tellurium atom; a $NR^{14}$ group in which the group $R^{14}$ represents a hydrogen atom, a straight-chain or branched-chain alkyl group having 1 to 18 carbon atoms which can be substituted by one or more carboxyl groups, phosphoryl groups or sulfonate groups, a substituted or unsubstituted aryl group which can comprise one or more heteroatoms, an aralkyl group, or a sulfonate group; or a $NOR^{14}$ group, wherein the group $R^{14}$ has the above definitions.

**4.** Use according to claim 3, wherein one or more ring atoms of the benzene nuclei of the compound having the general formula (I) are replaced by nitrogen atoms.

**5.** Use according to claim 3 or 4, wherein the compound having the general formula (I) is bound to a polyethyleneglycolester or a polyethyleneglycolether.

**6.** Use according to claim 2, wherein the isoindigo derivate is a compound having the general formula (II)

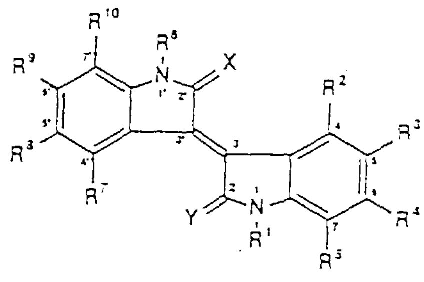

(II)

wherein $R^1$ to $R^{14}$ and X and Y have the meanings as defined in claim 3.

**7.** Use according to claim 6, wherein one or more ring atoms of the benzene nuclei of the compound having the general formula (II) are replaced by nitrogen atoms.

**8.** Use according to claim 6 or 7, wherein the compound having the general formula (II) is bound to a polyethyleneglycolester or a polyethyleneglycolether.

**9.** Use according to claim 2, wherein the indigo derivate is a compound having the general formula (III)

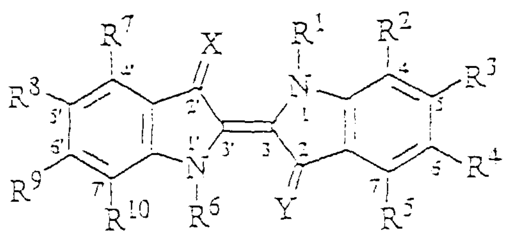

(III)

wherein $R^1$ to $R^{14}$ and X and Y have the meanings as defined in claim 3.

**10.** Use according to claim 9, wherein one or more ring atoms of the benzene nuclei of the compound having the general formula (III) are replaced by nitrogen atoms.

**11.** Use according to claim 9 or 10, wherein the compound having the general formula (III) is bound to a polyethyleneglycolester or a polyethyleneglycolether.

**12.** Use according to anyone of claims 1 to 11, wherein the indigoid bisindole derivative is in the form of a physiologically acceptable salt.

Fig. 1

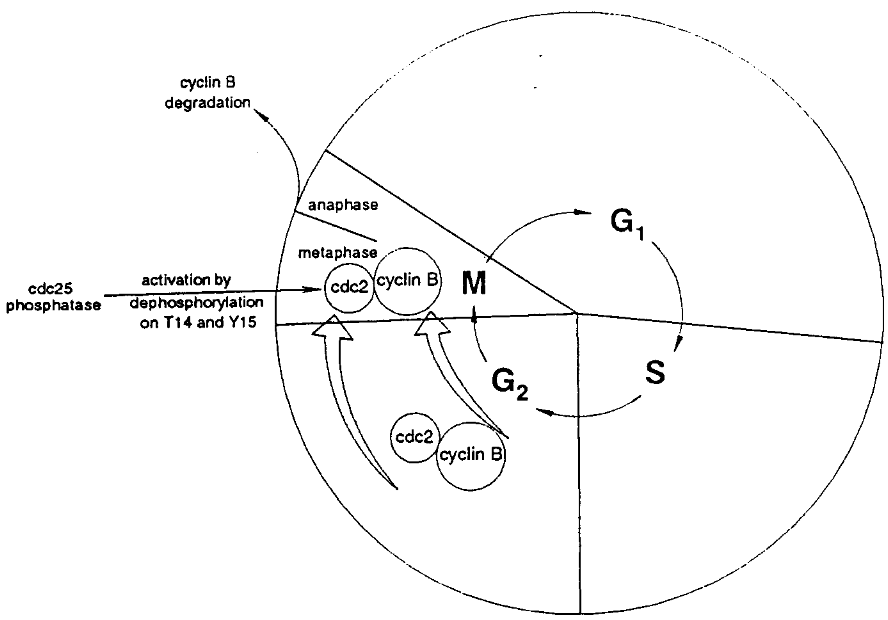
cyclin B
degradation
anaphase
metaphase
cdc25
phosphatase
activation by
dephosphorylation
on T14 and Y15
cdc2
cyclin B
M
G1
S
G2
cdc2
cyclin B

European Patent Office

# EUROPEAN SEARCH REPORT

Application Number
EP 98 10 9845

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | PATENT ABSTRACTS OF JAPAN vol. 010, no. 148 (C-350), 29 May 1986 & JP 61 007254 A (ISUKURA SANGYO KK), 13 January 1986 * abstract * | 1,2,9 | A61K31/40 |
| D,A | E.E. BROOKS ET AL.: "CVT-313, a specific and potent inhibitor of CDK2 that prevents neointimal proliferation." J. BIOL. CHEM., vol. 272, no. 46, 1997, pages 29207-29211, XP002079609 | | |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| THE HAGUE | 5 October 1998 | Klaver, T |

CATEGORY OF CITED DOCUMENTS

X particularly relevant if taken alone
Y particularly relevant if combined with another document of the same category
A technological background
O non-written disclosure
P intermediate document

T theory or principle underlying the invention
E earlier patent document, but published on, or after the filing date
D document cited in the application
L document cited for other reasons

& member of the same patent family, corresponding document

EPO FORM 1503 03 82 (P04C01)